# DEMANDE DE BREVET EUROPEEN

(11) **EP 0 524 093 A1**
(43) Date de publication de la demande: **20.01.1993**
(21) Numéro de dépôt: 92402046.4
(22) Date de dépôt: 16.07.1992
(51) Int. Cl.: C07D 305/14, A61K 31/335

(54) **Nouveaux dérivés d'analogues du taxol, leur préparation et les compositions qui les contiennent**

(30) Priorité: 16.07.1991 FR 9108937
(71) Demandeur: RHONE-POULENC RORER S.A., 92160 Antony (FR)
(72) Inventeur: Bourzat, Jean-Dominique, F-94300 Vincennes (FR); Commerçon, Alain, F-94400 Vitry sur Seine (FR); Guenard, Daniel, F-92120 Montrouge (FR); Gueritte-Voegelein, Françoise, F-91940 Les Ulis (FR); Potier, Pierre, F-75007 Paris (FR)
(74) Mandataire: Pilard, Jacques

(57) **Abrégé**

Nouveaux dérivés d'analogues du taxol de formule générale (I), leur préparation et les compositions qui les contiennent.

Dans la formule (I) :
- Ar représente un radical aryle,
- R représente un radical de formule générale

   R₇O- (II)

   dans laquelle R₇ représente un radical alcoyle éventuellement substitué, alcényle, alcynyle, cycloalcoyle, cycloalcényle, bicylcloalcoyle, phényle ou hétérocyclyle,
- R₁ et R₂, identiques ou différents, représentent un atome d'hydrogène ou un radical de formule générale (III) dans laquelle R₃ et R₄, identiques ou différents, représentent
   hydrogène ou alcoyle (éventuellement substitué par hydroxy, carboxy, alcoyloxycarbonyle ou un radical de formule (IV) dans laquelle R₅ et R₆, identiques ou différents, représentent hydrogène ou alcoyle ou forment ensemble un hétérocycle saturé ou non saturé à 5 ou 6 chaînons) ou bien R₃ et R₄ forment un hétéroycle saturé ou non saturé à 5 ou 6 chaînons.

Les nouveaux produits de formule (I) présentent des propriétés antitumorales et antileucémiques remarquables.

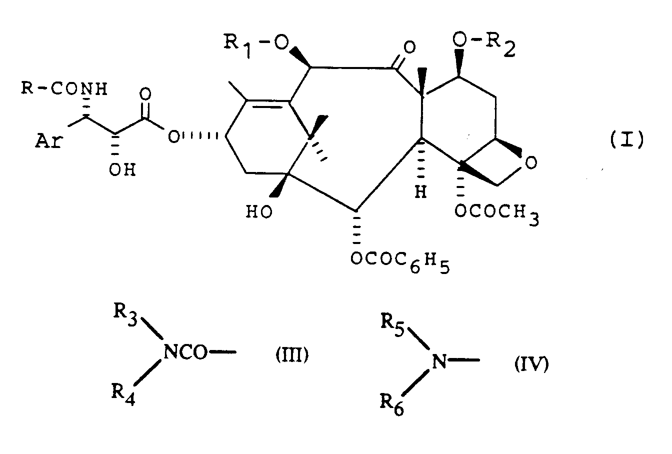

## Description

La présente invention concerne de nouveaux dérivés d'analogues du taxol de formule générale : 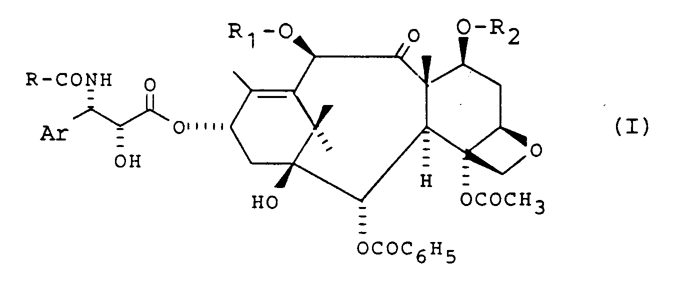
leur préparation et les compositions qui les contiennent.

Dans la formule générale (I),
Ar représente un radical aryle,
R représente
- un radical de formule générale

   R₇O- (II)

   dans laquelle R₇ représente un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en-4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 4 ou 6 chaînons et éventuellement substitué par un ou plusieurs radicaux alcoyles contenant 1 à 4 atomes de carbone,

étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₁ et R₂, identiques ou différents, représentent un atome d'hydrogène ou un radical de formule générale : 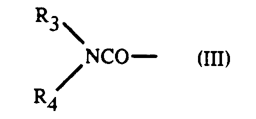
dans laquelle R₃ et R₄, identiques ou différents, représentent un atome d'hydrogène ou un radical alcoyle contenant 1 à 4 atomes de carbone en chaîne droite ou ramifiée éventuellement substitué :
a) par un radical hydroxy, carboxy, alcoyloxycarbonyle dont la partie alcoyle contient 1 à 4 atomes de carbone et est éventuellement substituée par un radical phényle,
b) par un radical de formule générale : 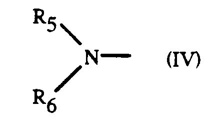 dans laquelle R₅ et R₆, identiques ou différents, représentent un atome d'hydrogène ou un radical alcoyle contenant 1 à 4 atomes de carbone en chaîne droite ou ramifiée, ou bien R₅ et R₆ forment ensemble avec l'atome d'azote auquel ils sont liés un hétérocycle saturé ou non saturé à 5 ou 6 chaînons contenant éventuellement un second hétéroatome choisi parmi les atomes d'azote (éventuellement substitué par un radical alcoyle contenant 1 à 4 atomes de carbone ou benzyle), d'oxygène ou de soufre, ou bien R₃ et R₄ forment ensemble avec l'atome d'azote auquel ils sont liés un hétérocycle saturé ou non saturé à 5 ou 6 chaînons contenant éventuellement un second hétéroatome choisi parmi les atomes d'azote (éventuellement substitué par un radical alcoyle contenant 1 à 4 atomes de carbone ou benzyle), d'oxygène ou de soufre,

étant entendu que l'un au moins des symboles R₁ ou R₂ représente un radical de formule générale (III).

De préférence Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et que les radicaux aryles sont des radicaux phényles ou α- ou β-naphtyles.

Plus particulièrement, Ar représente un radical phényle éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino).

Selon la présente invention, les nouveaux dérivés d'analogues du taxol de formule générale (I) peuvent être obtenus par action d'une amine de formule générale 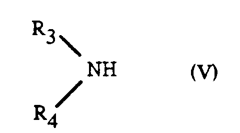
dans laquelle R₃ et R₄ sont définis comme précédemment sur un dérivé du taxane de formule générale: 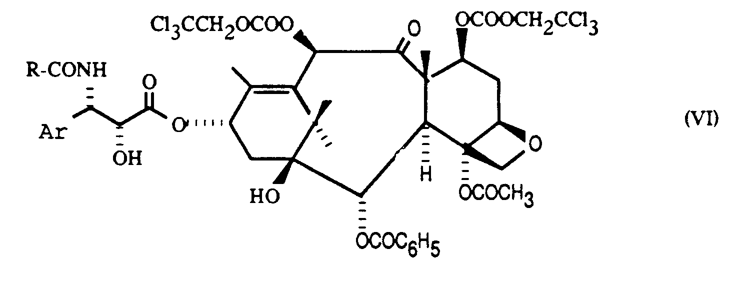
dans laquelle R et Ar sont définis comme précédemment, pour obtenir le produit de formule générale: 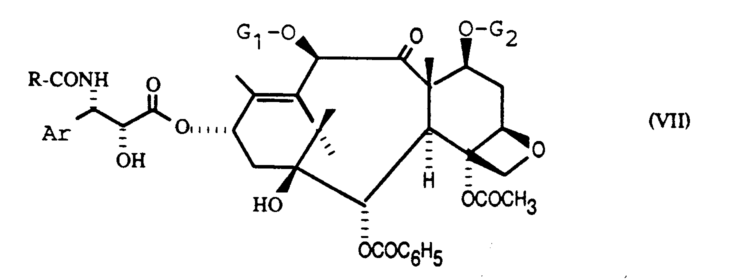
dans laquelle R et Ar sont définis comme précédemment, G₁ et G₂ représentent chacun un radical de formule générale (III) ou un groupement protecteur (CCl₃CH₂OCO-), étant entendu que l'un au moins des radicaux G₁ et G₂ représente un radical de formule générale (III), suivie, si nécessaire, du remplacement du ou des groupements protecteurs (CCl₃CH₂OCO-) par un atome d'hydrogène.

Généralement, l'action de l'amine de formule générale (V) sur le dérivé du taxane de formule (VI) est réalisée dans un solvant organique inerte tel qu'un hydrocarbure aliphatique halogéné comme le chlorure de méthylène à une température comprise entre 0°C et la température d'ébullition du mélange réactionnel. Afin d'éviter l'attaque en position -7, il est particulièrement avantageux d'opérer dans le chlorure de méthylène à une température inférieure à 50°C. En opérant à une température supérieure à 50°C, éventuellement en présence d'un excès suffisant d'amine de formule générale (V), il se forme un mélange des produits de formule générale (VII) dans laquelle l'un des radicaux G₁ ou G₂ représente un radical de formule générale (III) ou bien dans laquelle les deux radicaux G₁ et G₂ réprésentent chacun un radical de formule générale (III).

Le remplacement du groupement protecteur représenté par G₁ ou G₂ est généralement effectué par traitement par le zinc dans l'acide acétique éventuellement en présence de méthanol à une température comprise entre 30 et 80°C.

Les produits de formule générale (I) peuvent être séparés de leurs mélanges par chromatographie préparative sur des supports appropriés.

Le produit de formule générale (I) obtenu par le procédé selon l'invention peut être purifié par des méthodes physiques telles que la cristallisation ou la chromatographie sur un support approprié.

Le produit de formule générale (I) peut être transformé éventuellement en sel d'addition avec les acides minéraux (acides chlorhydrique, sulfurique, nitrique, phosphorique) ou organiques (acides acétique, oxalique, maléique, fumarique).

Les produits de formule générale (VI) peuvent être obtenus selon les procédés décrits dans les brevets européens EP-0 253 738 et EP-0 253 739.

Les produits de formule générale (I), et en particulier ceux pour lesquels R représente un radical t.butoxy, présentent des propriétés biologiques remarquables.

In vitro, la mesure de l'activité biologique est effectuée sur la tubuline extraite du cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R Acad. Sci., 293, Série II, 501-503 (1981). Dans cette étude les produits de formule générale (I) se sont montrés au moins aussi actifs que le taxol.

In vivo, les produits de formule générale (I) se sont montrés actifs chez la souris greffée par le mélanome B16 à des doses comprises entre 1 et 10 mg/kg par voie intrapéritonéale, ainsi que sur d'autres tumeurs liquides ou solides.

Par ailleurs, les produits de formule générale (I) ont une solubilité dans l'eau meilleure que celle du taxol ou des dérivés du taxane qui font l'objet du brevet européen EP-0 253 738.

Les exemples suivants illustrent la présente invention.

### EXEMPLE 1

Dans un ballon muni d'une agitation magnétique et d'un réfrigérant, on introduit 365 mg de t.butoxycarbonylamino-3 hydroxy-2 phényl-3 propionate-(2R,3S) d'acétoxy-4α benzoyloxy-2α époxy-5β,20 hydroxy- 1β oxo-9 bis-[(trichloro-2,2,2 éthoxy)carbonyloxyl-7β,10β taxène-11 yle-13α en solution dans 10 cm3 de chlorure de méthylène. On ajoute 80 µl de diméthylamino-3 propylamine. Le mélange réactionnel est chauffé à 40°C sous atmosphère d'argon pendant 4 heures. La solution est lavée par 2 fois 10 cm3 d'eau. Après séchage et évaporation du solvant, le résidu obtenu est purifié par chromatographie sur couche épaisse de silice en éluant avec un mélange chlorure de méthylène-méthanol (8-2 en volumes). On obtient ainsi 250 mg de t.butoxycarbonylamino-3 hydroxy-2 phényl-3 propionate-(2R,3S) d'acétoxy-4α benzoyloxy-2α époxy-5β,20 hydroxy-1β oxo-9 (diméthylamino-3 propyl)amicarbonyloxy-10β (trichloro-2,2,2 éthoxy)carbonyloxy]-7β taxène-11 yle-13α dont la structure est confirmée par le spectre de résonance magnétique nucléaire du proton déterminé dans le chloroforme deutéré, les déplacements chimiques étant exprimés en ppm et les constantes de couplage (J) en Hertz:
1,16 (s, 3H) ; 1,25 (s, 3H) ; 1,35 (s, 9H) ; 1,83 (s, 3H) ; 1,93 (s, 3H) ; 2,26 (s, 6H) ; 2,38 (s, 3H) ; 3,25 (m, 2H) ; 3,93 (d, J=7, 1H) ; 4,16 et 4,33 (2d, J=9, 2H) ; 4,62 (s, large, 1H) ; 4,73 et 4,99 (2d, J=12, 2H) ; 4,96 (d, J=9, 1H) ; 5,29 (d, J=9, 1H) ; 5,47 (d, J=9, 1H) ; 5,54 (m, 1H) ; 5,66 (d, J=7, 1H) ; 6,19 (t, J=9, 1H) ; 6,29 (s, 1H) ; 7,37 (5H) ; 7,49, 7,62 et 8,09 (5H).

A une solution de 137 mg du produit obtenu précédemment dans un mélange acide actique-méthanol (1-1 en volumes), on ajoute 130 mg de zinc en poudre. Le mélange réactionnel est agité à 60°C pendant 1 heure. Après filtration et concentration à sec, le résidu est repris par de l'eau puis extrait par de l'acétate d'éthyle. Après décantation et séchage, les phases organiques sont concentrées à sec. Le résidu obtenu est purifié par chromatographie en couche épaisse de silice en éluant avec un mélange chlorure de méthylène-méthanol (8-2 en volumes). On obtient ainsi 73 mg de t.butoxycarbonylamino-3 hydroxy-2 phényl-3 propionate-(2R,3S) d'acétoxy-4α benzoyloxy-2α époxy-5β,20 dihydroxy-1β,7β oxo-9 (diméthylamino-3 propyl) aminocarbonyloxy-10β taxène-11 yle-13α qui présente les caractéristiques suivantes:
- spectre ultra-violet (éthanol) :
   λ max = 230 nm (ε = 12700)
   λ max = 275 nm (ε = 1040)
- spectre infra-rouge (en solution dans le chlorure de méthylène) : principales bandes d'absorption caractéristiques à 3400, 2960 et 1729 cm⁻¹
- spectre de résonance magnétique nucléaire du proton:
   1,18 (s, 3H) ; 1,23 (s, 3H) ; 1,33 (s, 9H) ; 1,66 (s, 3H) ; 1,87 (s, 3H) ; 2,28 (s, 6H) ; 2,38 (s, 3H) ; 3,20 (m, 2H) ; 3,78 (d, J=7, 1H) ; 4,17 et 4,31 (2d, J=8, 2H) ; 4,43 (dd, J=6 et 12, 1H) ; 4,62 (s large, 1H) ; 4,97 (d, J=9, 1H) ; 5,26 (d, J=9, 1H) ; 5,54 (d, J=9, 1H) ; 5,66 (d, J=7, 1H) ; 6,21 (m, 2H) ; 7,39 (5H) ; 7,51, 7,62 et 8,11 (5H)
- spectre de masse (FAB): 936 (MH⁺)

A une solution du produit obtenu précédemment dans 0,5 cm3 d'éthanol, on ajoute 0,780 cm3 d'une solution d'acide chlorhydrique 0,1M. Le mélange réactionnel est concentré à sec puis lyophilisé. On obtient ainsi le chlorhydrate du t.butoxycarbonylamino-3 hydroxy-2 phényl-3 propionate-(2R,3S) d'acétoxy-4α benzoyloxy-2α époxy-5β,20 dihydroxy-1β,7β oxo-9 (diméthylamino-3 propyl) aminocarbonyloxy-10β taxène-11 yle-13α dont les caractéristiques sont les suivantes:
- pouvoir rotatoire: [α]²⁰_{D} = -29° (c = 0,4; éthanol)
- spectre de résonance magnétique nucléaire du proton (CDCl₃-CD₃OD) :
   1,16 (s, 3H) ; 1,21 (s, 3H) ; 1,40 (s, 9H) ; 1,66 (s, 3H) ; 1,93 (s, 3H) ; 2,38 (s, 3H) ; 2,83 (s, 6H) ; 3,16 (m, 2H) ; 3,81 (d, J=7, 1H) ; 4,23 et 4,31 (2d, J=8, 2H) ; 4,38 (m, 1H) ; 4,58 (m, 1H) ; 4,99 (d, J=9, 1H) ; 5,16 (s, 1H) ; 5,66 (d, J=7, 1H) ; 6,16 (t, J=9, 1H) ; 6,26 (s, 1H) ; 7,37 (5H) ; 7,52, 7,70 et 8,09 (5H).

### EXEMPLE 2

A une solution de 4,63 g de tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α dans 100 cm3 d'acétonitrile, maintenue sous atmosphère d'argon, on ajoute 0,50 cm3 de diméthylamino-3 propylamine. Le milieu réactionnel est chauffé sous agitation pendant 3 heures à une température voisine de 60°C puis réfroidi à une température voisine de 20°C et concentré à sec sous pression réduite (2,7 kPa) à 40°C. On obtient 5,3 g d'une meringue blanche que l'on purifie par chromatographie sur 150 g de silice (0,063-0,2 mm) contenus dans une colonne de 4 cm de diamètre [éluant : dichlorométhaneméthanol (95-5 en volumes)] en recueillant des fractions de 100 cm3. Les fractions 1 à 10 sont éliminées, puis on poursuit la chromatographie en éluant avec un mélange : dichlorométhane-méthanol (80-20 en volumes). Les fractions 17 à 30 sont réunies et concentrées à sec sous pression réduite (2,7 kPa) à une température voisine de 40°C. On obtient ainsi 2,57 g d'un mélange en proportions molaires : 66/33 des deux dérivés de substitution, [(diméthylamino-3 propyl) carbamoyloxy]-, respectivement en position 10 et 7.

Le mélange précédent est séparé par chromatographie liquide haute performance sur 400 g de support, dont la préparation est décrite ci-après, contenu dans une colonne de 25 cm de longueur et de 6 cm de diamètre avec comme phase mobile le mélange hexane-éthanol (80-20-2,5 en volumes) au débit de 45 cm3/minute. On obtient par ordré d'élution successive :
- 0,46 g de tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxy]-7β oxo-9 (trichloro-2,2,2 éthoxy) carbonyloxy-10β taxène-11 yle-13α sous forme d'une meringue blanche,
- 0,81 g de tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxy]-10β oxo-9 (trichloro-2,2,2 éthoxy) carbonyloxy-7β taxène-11 yle-13α sous forme d'une meringue blanche.

Le support peut être préparé de la manière suivante:
Dans un tricol de 6 litres, on met en suspension 600 g de silice aminopropyle (100 Å - 10 µm - NH₂ ; Macherey-Nagel) dans 2 litres de diméthylformamide. On ajoute 95 g d'anhydride de l'acide N-tert-butoxycarbonylamino-11 undécanoïque et on agite le mélange réactionel pendant 18 heures à une température voisine de 20°C. La silice est séparée par filtration et lavée successivement par deux fois 1500 cm3 de dichlorométhane puis deux fois 1500 cm3 de diméthylformamide. La silice ainsi lavée est remise en suspension dans 2 litres de diméthylformamide et on ajoute 95 g d'anhydride de l'acide N-tert-butoxycarbonylamino-11 undécanoïque puis on agite le mélange réactionnel pendant 18 heures à une température voisine de 20°C.

La silice est séparée par filtration, lavée successivement par deux fois 600 cm3 de dichlorométhane, deux fois 600 cm3 de tétrahydrofuranne, deux fois 600 cm3 de méthanol et deux fois 600 cm3 d'oxyde de diéthyle puis séchée sous pression réduite à une température voisine de 20°C. On obtient ainsi 610 g de silice désignée par l'appellation "BOC-C₁₁-C₃-silice" sous forme de poudre blanche dont la structure est confirmée par le spectre infra-rouge et dont l'analyse élémentaire (trouvée) est : C% = 8,8 ; H% = 1,7 ; N% = 1,2.

Dans un tricol de 6 litres, on met en suspension 607 g de silice "BOC-C₁₁-C₃-silice" dans 2 litres de dichlorométhane et 69 cm3 de pyridine. On ajoute goutte à goutte 530 cm3 de diméthyloctylchlorosilane puis on agite le mélange réactionnel pendant 16 heures à une température voisine de 20°C. Le solide obtenu est séparé par filtration et lavé successivement par deux fois 1 litre de dichlorométhane, deux fois 1 litre de méthanol, deux fois 1 litre de tétrahydrofuranne, deux fois 1 litre de dichlorométhane et deux fois 1 litre d'oxyde de diéthyle puis séché sous pression réduite à une température voisine de 20°C. On obtient ainsi 712 g de silice désignée par l'appellation "BOC-C₁₁-C₃-silice-O-Si(CH₃)₂(CH₂)₇CH₃" sous forme de poudre blanche dont la structure est confirmée par le spectre infra-rouge et dont l'analyse élémentaire (trouvée) est: C % = 12,1; H % = 2,4; N % = 1,0.

Dans un tricol de 6 litres, on met en suspension 711 g de silice "BOC-C₁₁-C₃-silice-O-Si(CH₃)₂(CH₂)₇CH₃" dans 2200 cm3 d'une solution à 6 % en volumes d'acide trifluoroacétique dans le dichlorométhane. On agite le mélange réactionnel pendant 5 heures à une température voisine de 20°C. La silice est séparée par filtration et lavée successivement par deux fois 1 litre de dichlorométhane, deux fois 1 litre d'un mélange dichlorométhane/diisopropyléthylamine (70/30 en volumes), 1 litre de dichlorométhane, deux fois 1 litre de tétrahydrofuranne, deux fois 1 litre de méthanol et deux fois 1 litre d'oxyde de diéthyle puis séchée sous pression réduite à une température voisine de 50°C. La silice ainsi lavée et séchée est remise en suspension dans 2 litres d'une solution à 6 % en volumes d'acide trifluoroacétique dans le dichlorométhane.

On agite le mélange réactionnel pendant 16 heures à une température voisine de 20°C. La silice est séparée par filtration et lavée successivement par deux fois 1,5 litre de dichlorométhane, deux fois 1 litre d'un mélange dichlorométhane/diisopropyléthylamine (70/30 en volumes), 1,5 litre de dichlorométhane, deux fois 2 litres de tétrahydrofuranne, deux fois 2 litres de méthanol et deux fois 2 litres d'oxyde de diéthyle puis séchée sous pression réduite à une température voisine de 50°C. On obtient ainsi 607 g de silice désignée par l'appellation "C₁₁-C₃-silice-O-Si(CH₃)₂(CH₂)₇CH₃" sous forme de poudre blanche dont la structure est confirmée par le spectre infra-rouge et dont l'analyse élémentaire (trouvée) est : C % = 8,8 ; H % = 1,7 ; N % = 1,3.

Dans un tricol de 4 litres, on met en suspension 400 g de silice "C₁₁-C₃-silice-O-Si(CH₃)₂(CH₂)₇CH₃" dans 1800 cm3 de diméthylformamide. On ajoute 42 g de dinitro-3,5 benzoyl-D-phénylglycine et 30 g d'éthoxy-2 éthoxycarbonyl-1 dihydro-1,2 quinoléine et on agite le mélange réactionnel pendant 16 heures à une température voisine de 20°C. La silice est séparée par filtration et lavée successivement par deux fois 1 litre de dichlorométhane, deux fois 1 litre de tétrahydrofuranne, deux fois 1 litre de méthanol et deux fois 1 litre d'oxyde de diéthyle. La silice ainsi lavée est remise en suspension dans 2 litres de diméthylformamide et on ajoute 30 g d'éthoxy-2 éthoxycarbonyl-1 dihydro-1,2 quinoléine et 42 g de dinitro-3,5 benzoyl-D-phénylglycine puis on agite le mélange réactiomel pendant 5 heures à une température voisine de 20°C. La silice est séparée par filtration, lavée successivement par deux fois 1 litre de diméthylformamide, deux fois 1 litre de dichlorométhane, deux fois 1 litre de tétrahydrofuranne, deux fois 1 litre de méthanol et deux fois 1 litre d'oxyde de diéthyle puis séchée sous pression réduite à une température voisine de 140°C. On obtient ainsi 434 g de silice désignée par l'appellation "DNB-D-Phg-C₁₁-C₃-silice-O-Si(CH₃)₂(CH₂)₇CH₃" sous forme de poudre blanche dont la structure est confirmée par le spectre infra-rouge et dont l'analyse élémentaire (trouvée) est: C % = 12,3; H % = 1,8; N % = 2,1.

Dans un tricol de 4 litres, on met en suspension 434 g de silice "DNB-D-Phg-C₁₁-C₃-silice-O-Si(CH₃)₂(CH₂)₇CH₃" dans 1,3 litre de dichlorométhane et on ajoute 100 cm3 de diméthyloctylméthoxysilane puis on agite le mélange réactionnel pendant 54 heures à une température voisine de 20°C. La silice est séparée par filtration, lavée successivement par deux fois 1 litre de dichlorométhane, deux fois 1 litre de méthanol, deux fois 1 litre de tétrahydrofuranne et deux fois 1 litre de dichlorométhane puis séchée sous pression réduite à une température voisine de 140°C. On obtient ainsi 425 g de silice désirée par l'appellation "DNB-D-Phg-C₁₁-C₃-silice-O-Si(CH₃)₂(CH₂)₇CH₃ réoctylée" sous forme de poudre blanche dont la structure est confirmée par le spectre infra-rouge et dont l'analyse élémentaire (trouvée) est: C % = 12,7; H % = 1,9; N % = 2,0.

Dans un tricol de 4 litres, on met en suspension 425 g de silice "DNB-D-Phg-C₁₁-C₃-silice-OSi(CH₃)₂(CH₂)₇CH₃ réoctylée" 1,3 litre de dichlorométhane. On ajoute goutte à goutte 545 cm3 de triméthylsilylimidazole et on agite le mélange réactionnel pendant 15 heures à une température voisine de 20°C. Le solide obtenu est séparé par filtration et lavé successivement par deux fois 1 litre de tétrahydrofuranne, deux fois 1 litre de méthanol, deux fois 1 litre d'acétone et deux fois 1 litre de dichlorométhane puis séché sous pressions réduite à une température voisine de 20°C. On obtient ainsi 431 g de silice désignée par l'appellation "DNB-D-Phg-C₁₁-C₃-silice-[O-Si(CH₃)₂(CH₂)₇-CH₃]" sous forme de poudre blanche dont la structure est confirmée par le spectre infra-rouge et dont l'analyse élémentaire (trouvée) est: C %= 13,7; H % = 2,2 ; N % = 2,0.

L'anhydride de l'acide N-tert-butoxycarbonylamino-11 undécanoïque peut être préparé de la manière suivante :
A une solution de 30,1 g d'acide N-tert-butoxycarbonylamino-11 undécanoïque dans 480 cm3 d'acétate d'éthyle, maintenue à une température voisine de 5°C, on ajoute en 10 minutes une solution de 10,63 g de N,N′-dicyclohexylcarbodiimide dans 120 cm3 d'acétate d'éthyle. Le mélange réactionnel est agité pendant 1 heure à une température voisine de 5°C puis pendant 16 heures à une températue voisine de 20°C. Le pécipité est séparé par filtration et lavé par 30 cm3 d'acétate d'éthyle. Le filtrat est concentré sous pression réduite à 30°C. Le solide obtenu est séché sous vide à une température voisine de 30°C. On obtient ainsi 31 g d'anhydride de l'acide N-tert-butoxycarbonylamino-11 undécanoïque fondant à 58°C.

L'acide N-tert-butoxycarbonylamino-11 undécanoïque peut être préparé selon la méthode décrite par J.T. SPARROW, J. Org. Chem., 41,1350 (1976).

Une solution de 104 mg de tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propryl) carbamoyloxy]-10β oxo-9 (trichloro-2,2,2 éthoxy) carbonyloxy -7β taxène-11 yle-13α précédemment obtenu, dans un mélange de 8 cm3 de méthanol et de 8 cm3 d'acide acétique est chauffée sous agitation et sous atmosphère d'argon jusqu'à une température voisine de 60°C puis additionnée de 1,2 g de zinc en poudre. Le mélange réactionnel est ensuite agité pendant 1 heure 30 minutes à 60°C puis refroidi à une températue voisine de 20°C et filtré sur verre fritté garni de célite. le verre fritté est lavé par 3 fois 5 cm3 de dichlorométhane et les filtrats sont réunis puis concentrés à sec sous pression réduite (2,7 kPa) à une température voisine de 40°C.

Le résidu est additionné de 5 cm3 d'eau et de 5 cm3 d'acétate d'éthyle. La phase aqueuse est séparée par décantation puis réextraite par 3 fois 5 cm3 d'acétate d'éthyle. Les phases organiques sont réunies, séchées sulfate de magnésium, filtrées puis concentrées à sec sous pression réduite (2,7 kPa) à 40°C. On obtient 19 mg de tert-butoxycarbonylamino-3 hydroxy-2 phényl-3 propionate-(2R,3S) d'acétoxy-4 benzoloxy-2α époxy-5β,20 dihydroxy-1,7β [(diméthylamino-3 propyl) carbamoyloxy]-10β oxo-9 taxène-11 yle-13α sous forme d'une meringue blanche dont les caractéristiques sont les suivantes :
- spectre de RMN (400 MHz ; CDCl₃)
δ (ppm) : 1,30 (2s, 3H chacun : -CH₃ 16 et 17) ; 1,38 (s, 9H : -C(CH₃)₃) ; 1,70 (s, 3H : -CH₃ 18 ou 19) ; 1,90 (s, 3H : -CH₃ 18 ou 19) ; 1,90 [m, 2H : -NHCH₂CH₂CH₂N(CH₃)₂] ; 2,2 à 2,90 [m, 15H : -(CH₂)- 6, -CH₂- 14, -CH₂N(CH₃)₂, -N(CH₃)₂, -COCH₃] ; 3,2 et 3,4 [2m, 2H : -NHCH₂CH₂CH₂N(CH₃)₂]; 3,8 (d, 1H : -H 3) ; 4,2 et 4,34 (AB, 2H : -CH₂- 20) ; 4,45 (dd, 1H, J = 11 et 7 : -H 7) ; 4,66 (bs, 1H : -H 2′) ; 5,0 (bd, 1H; J = 9 : -H 5) ; 5,3 (bs, 1H : -H 3′) ; 5,6 [bd, 1H : -NHCH₂CH₂CH₂N(CH₃)₂] ; 5,68 (d, 1H, J = 7 : -H 2) ; 6,25 (m, 2H : -H 13 et -H 10) ; 6,4 [bs, 1H : -NHCOOC(CH₃)₃] ; 7,2 à 7,4 (mt, 5H : -C₆H₅ 3′); 7,5 [t, 2H, J = 7,5 : -OCOC₆H₅ (-H 3 et -H 5)] ; 7,64 [t, 1H : -OCOC₆H₅ (-H 4)] ; 8,12 [d, 2H : -OCOC₆H₅ (-H 2 et -H 6)].

Une solution de 59 mg de tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxyl-7β oxo-9 (trichloro-2,2,2 éthoxy) carbonyloxy-10β taxène-11 yle-13α précédemment obtenu dans un mélange de 6 cm3 de méthanol et de 6 cm3 d'acide acétique est chauffée sous agitation et sous atmosphère d'argon jusqu'à une température voisine de 60°C puis additionnée de 1,2 g de zinc en poudre. Le mélange réactionnel est ensuite agité pendant 1 heure 30 minutes à 60°C puis refroidi à une température voisine de 20°C et filtré sur verré fritté garni de célite. Le verre fritté est lavé par 3 fois 5 cm3 de dichlorométhane et les filtrats sont réunis puis concentrés à sec sous pression réduite (2,7 kPa) à une température voisine de 40°C. Le résidu est additionné de 5 cm3 d'eau et de 5 cm3 d'acétate d'éthyle. La phase aqueuse est séparée par décantation puis réextraite par 3 fois 5 cm3 d'acétate d'éthyle. Les phases organiques sont réunies, séchées sur sulfate de magnésium, filtrées puis concentrées à sec sous pression réduite (2,7 kPa) à 40°C. On obtient 9 mg de tert-butoxycarbonylamino-3 hydroxy-2 phényl-3 propionate-(2R,3S) d'acétoxy-4 benzoyloxy-2α époxy-5β,20 dihydroxy-1,10β [(diméthylamino 3 propyl) carbamoyloxyl-7β oxo-9 taxène-11 yle-13α sous forme d'une meringue blanche dont les caractéristiques sont les suivantes :
- spectre de RMN (400 MHz ; CDCl₃)
δ (ppm) : 1,30 (2s, 3H chacun : -CH₃ 16 et 17) ; 1,36 (s, 9H : -C(CH₃)₃) ; 1,82 (s, 3H : -CH₃ 18 ou 19) ; 1,95 (s, 3H : -CH₃ 18 ou 19) ; 1,88 [m, 2H : -NHCH₂CH₂CH₂N(CH₃)₂] ; 2,2 à 2,70 [m, 15H : -(CH₂)- 6, -CH₂- 14, -CH₂N(CH₃)₂, -N(CH₃)₂, -COCH₃] ; 3,15 et 3,3 [2m, 2H : -NHCH₂CH₂CH₂N(CH₃)₂]; 4,0 (d, 1H : -H 3) ; 4,2 et 4,38 (AB, 2H : -CH₂- 20) ; 4,68 (bs, 1H : -H 2′) ; 4,96 (bd, 1H; J = 9 Hz : -H 5) ; 5,3 (bs, 1H : -H 3′) ; 5,40 (dd, 1H, J = 11 et 7 : -H 7) ; 5,5 [s+ bs, 2H : -H 10 et -NHCH₂CH₂CH₂N(CH₃)₂] ; 5,7 [d + bs, 2H : -H 2 et -NHCOOC(CH₃)₃] ; 6,22 (bt, 1H : -H 13) ; 7,2 à 7,4 (mt, 5H : -C₆H₅ 3′); 7,53 [t, 2H, J = 7,5 : -OCOC₆H₅ (-H 3 et -H 5)] ; 7,65 [t, 1H : -OCOC₆H₅ (-H 4)] ; 8,12 [d, 2H : -OCOC₆H₅ -H 2 et -H 6)].

### EXEMPLE 3

En opérant d'une manière analogue à celle décrite à l'exemple 2, mais à partir de 6,95 g de tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α et de 0,94 g de (méthyl-4 pipérazinyl)-3 propylamine, on obtient après purification par chromatographie liquide haute performance avec comme phase mobile le mélange méthanol-éthanol-hexanedichlorométhane (10-10-80-2 en volumes) :
- 1,37 g de tert-butoxycarbonylamino-3 hydroxy-2 phényl-3 propionate- (2R,3S) d'acétoxy-4 benzoyloxy-2α époxy-5β,20 hydroxy-1 {[(méthyl-4 pipérazinyl)-3 propyl] carbamoyloxy}-10β oxo-9 (trichloro-2,2,2 éthoxy) carbonyloxy-7β taxène-11 yle-13α sous forme d'une meringue blanche,
2,17 g de tert-butoxycarbonylamino-3 hydroxy-2 phényl-3 propionate-(2R,3S) d'acétoxy-4 benzoyloxy-2α époxy-5β,20 hydroxy-1 {[(méthyl-4 pipérazinyl)-3 propyl] carbamoyloxy}-7β oxo-9 (trichloro-2,2,2 éthoxy) carbonyloxy-10β taxène-11 yle-3α sous forme d'une meringue blanche.

Le tert-butoxycarbonylamino-3 hydroxy-2 phényl-3 propionate-(2R,3S) d'acétoxy-4 benzoyloxy-2α époxy-5β,20 hydroxy-1 {[(méthyl-4 pipérazinyl)-3 propyl] carbamoyloxy}-10β oxo-9 (trichloro-2,2,2 éthoxy) carbonyloxy-7β taxène-11 yle-13α est transformé en tert-butoxycarbonylamino-3 hydroxy-2 phényl-3 propionate-(2R,3S) d'acétoxy-4 benzoyloxy-2α époxy-5β,20 hydroxy-1,7β {[(méthyl-4 pipérazinyl)-3 propyl] carbamoyloxy}-10β oxo-9 taxène-11 yle-13α par action du zinc dans un mélange de méthanol et d'acide acétique comme décrit à l'exemple 2 pour le tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxyl-10β oxo-9 (trichloro-2,2,2 éthoxy) carbonyloxy-7β taxène-11 yle-13α.

Le produit ainsi obtenu possède les caractéristiques suivantes :
- spectre de RMN (400 MHz; CDCl₃)
δ (ppm) : 1,18 (s, 3H : -CH₃ 16 et 17) ; 1,27 (s, 3H : -CH₃ 16 ou 17) ; 1,33 (s, 9H : -C(CH₃)₃) ; 1,7 (s, 3H : -CH₃ 19) ; 1,6 à 1,95 [mt, 3H : -OCONHCH₂CH₂CH₂N= et -(CH)-H 6] ; 1,88 (s, 3H : -CH₃ 18) ; 2,2 à 2,7 [mt, 13H : -CH₂N(CH₂CH₂)₂NCH₃, -CH₂- 14 et -(CH)- H 6] ; 2,29 (s, 3H : =NCH₃) ; 2,4 (s, 3H, : -COCH₃) ; 3,2 à 3,45 (mt, 2H : -OCONHCH₂CH₂CH₂N=) ; 3,8 (d, 1H, J = 7 : -H 3) ; 4,18 et 4,31 (2d, 1H chacun, J = 8 : -CH₂- 20) ; 4,44 (dd, 1H; J = 11 et 7 : -H 7) ; 4,63 (mf, 1H : - H 2′) ; 4,97 (d large, 1H, J = 10 : -H 5) ; 5,27 (d large, 1H, J = 9,5 : -H 3′) ; 5,4 [d, 1H, J = 9,5 : -NHCOOC(CH₃)₃] ; 5,67 (d, 1H, J = 7 : -H 2) ; 6,20 (s, 1H : -H 10) ; 6,25 (mt, 1H : -H 13) ; 7,0 (mf, 1H : -NHCH₂CH₂CH₂N=) ; 7,25 à 7,45 (mt, 5H : -C₆H₅ 3′) ; 7,51 [t, 2H, J = 8 : -OCOC₆H₅(-H 3 et -H 5)] ; 7,62 [t, 1H, J = 8 : -OCOC₆H₅ (-H 4)] ; 8,13 [d, 2H, J = 8 : -OCOC₆H₅ (-H 2 et -H 6)].

A une solution de 5 mg de tert-butoxycarbonylamino-3 hydroxy-2 phényl-3 propionate-(2R,3S) d'acétoxy-4 benzoyloxy-2α époxy-5β,20 dihydroxy-1,7β-{[(méthyl-4 pipérazinyl)-3 propyl] carbamoyloxy}-10β oxo-9 taxène-11 yle-13α obtenus précédemment, dans 0,1 cm3 d'une solution aqueuse 0,1N d'acide chlorhydrique, on ajoute 0,2 cm3 d'eau distillée et lyophilise la solution obtenue. On obtient ainsi 5 mg de dichlorhydrate de tert-butoxycarbonylamino-3 hydroxy-2 phényl-3 propionate-(2R,3S) d'acétoxy-4 benzoyloxy-2α époxy-5β,20 dihydroxy-1,7β-{[(méthyl-4 pipérazinyl)-3 propyl] carbamoyloxy}-10β oxo-9 taxène-11 yle-13α dont les caractéristiques sont les suivantes :
- spectre de RMN (400 MHz; D₂O + ε CD₃COOD)
δ (ppm) : 0,88 (s, 3H : -CH₃ 16 et 17) ; 0,93 (s, 3H : -CH₃ 16 ou 17) ; 1,09 (s, 9H : -C(CH₃)₃) ; 1,4 (s, 3H : -CH₃ 19) ; 1,4 à 1,9 (mt, 5H : -OCONHCH₂CH₂CH₂N=, -CH₂- 14 et -(CH)-H 6) ; 1,66 (s, 3H : -CH₃ 18) ; 2,09 (s, 3H : -COCH₃) ; 2,32 (mt, 1H : -(CH)-H 6) ; 2,81 (s, 3H : -NCH₃) ; 2,9 à 3,2 et 3,4 à 3,55 (2mt respectivement 4H et 9H : -OCONHCH₂CH₂CH₂N(CH₂CH₂)₂NCH₃ et -H 3) ; 4,01 (mt, 3H : -CH₂- 20 et -H 7) ; 4,36 (d, 1H, J = 6,6 Hz : -H 2′) ; 4,6 à 4,8 (-H 3′ : signal annulé par la présaturation du signal du solvant) ; 4,84 (d large, 1H, J = 9 : -H 5) ; 5,34 (d, 1H, J = 7 : -H 2) ; 5,82 (t, 1H, J = 9 : -H 13) ; 6,01 (s, 1H : -H 10) ; 7,03 [t, 1H, J = 8 : -C₆H₅ 3′ (-H 4)] ; 7,13 [d, 2H, J = 8 : C₆H₅ 3′ (-H 2 et -H 6)] ; 7,2 [t, 2H, J = 8 : -C₆H₅ 3′ (-H 3 et -H 5)] ; 7,38 [t, 2H, J = 8 : -OCOC₆H₅(-H 3 et -H 5)] ; 7,51 [t, 1H, J = 8 : -OCOC₆H₅(-H 4)] ; 7,8 [d, 2H, J = 8 : -OCOC₆H₅ (-H 2 et -H 6)].

Le tert-butoxycarbonylamino-3 hydroxy-2 phényl-3 propionate- (2R,3S) d'acétoxy-4 benzoyloxy-2α époxy-5β,20 hydroxy- 1 {[(méthyl-4 pipérazinyl)-3 propyl] carbamoyloxy}-7β oxo-9 (trichloro-2,2,2 éthoxy) carbonyloxy-10β taxène-11 yle-13α est transformé en tert-butoxycarbonylamino-3 hydroxy-2 phényl-3 propionate-(2R,3S) d'acétoxy-4 benzoyloxy-2α époxy-5β,20 dihydroxy-1,10β {[(méthyl-4 pipérazinyl)-3 propyl] carbamoyloxy}-7β oxo-9 taxène-11 yle-13α par action du zinc dans un mélange de méthanol et d'acide acétique comme décrit à l'exemple 2 pour le tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxyl]-10β oxo-9 (trichloro-2,2,2 éthoxy) carbonyloxy-7β taxène-11 yle-13α.

Le produit ainsi obtenu possède les caractéristiques suivantes :
- pouvoir rotatoire : [α]²⁰_{D} = -14° (c = 0,41 ; méthanol)
- spectre de RMN (400 MHz; CDCl₃)
   δ (ppm) : 1,13 (s, 3H : -CH₃ 16 ou 17) ; 1,24 (s, 3H : -CH₃ 16 ou 17) ; 1,37 (s, 9H : -C(CH₃)₃) ; 1,6 à 2,0 [mt, 3H : -OCONHCH₂CH₂CH₂N= et -(CH)-H 6] ; 1,84 (s, 3H : -CH₃ 19) ; 1,92 (s, 3H : -CH₃ 18) ; 2,3 (d, 2H, J = 8,5 : -CH₂- 14) ; 2,38 (s, 6H : =NCH₃ et -COCH₃) ; 2,3 à 2,8 [mt, 11H : -CH₂N(CH₂CH₂)₂NCH₃ et -(CH)-H 6] ; 3,14 et 3,3 (2 mt, 1H chacun: -OCONHCH₂CH₂CH₂N=) ; 4,01 (d, 1H, J = 7 : -H 3) ; 4,21 et 4,33 (2d, 1H chacun, J = 9 : -CH₂- 20) ; 4,63 (s large, 1H : -H 2′) ; 4,94 (d large, 1H, J = 10 : -H 5) ; 5,27 (d large, 1H, J = 9,5 : -H 3′) ; 5,38 (dd, 1H, J = 11 et 7 : -H 7); 5,44 [d, 1H, J = 9,5 : -NHCOOC(CH₃)₃] ; 5,51 (s, 1H: -H 10) ; 5,68 (d, 1H, J = 7 : -H 2) ; 6,09 (mf, 1H : -NHCH₂CH₂CH₂N=) ; 6,21 (t large, 1H, J = 8,5 : -H 13) ; 7,25 à 7,45 (mt, 5H : -C₆H₅ 3′) : 7,50 [t, 2H, J = 8 : -OCOC₆H₅(-H 3 et -H 5)] ; 7,62 [t, 1H, J = 8: -OCOC₆H₅(-H 4)] ; 8,10 [d, 2H, J = 8 : -OCOC₆H₅(-H 2 et -H 6)].

A une solution de 5 mg de tert-butoxycarbonylamino-3 hydroxy-2 phényl-3 propionate-(2R,3S) d'acétoxy-4 benzoyloxy-2α époxy-5β,20 dihydroxy-1,10β {[(méthy1-4 pipérazinyl)-3 propyl] carbamoyloxy}-7β oxo-9 taxène-11 yle-13α précédemment obtenus, dans 0,1 cm3 d'une solution aqueuse 0,1N d'acide chlorhydrique on ajoute 0,3 cm3 d'eau distillée et lyophilise la solution obtenue. On obtient ainsi 5 mg de dichlorhydrate de tert-butoxycarbonylamino-3 hydroxy-2 phényl-3 propionate-(2R,3S) d'acétoxy-4 benzoyloxy-2α époxy-5β,20 dihydroxy- 1,10β {[(méthy1-4 pipérazinyl)-3 propyl] carbamoyloxy}-7β oxo-9 taxène-11 yle-13α dont les caractéristiques sont les suivantes :
- spectre de RMN (400 MHz; D₂O + ε CD₃COOD)
δ (ppm) : 0,82 (s, 3H : -CH₃ 16 ou 17) ; 0,92 (s, 3H : -CH₃ 16 ou 17) ; 1,1 (s, 9H : -C(CH₃)₃) ; 1,4 à 1,8 (mt, 5H: -OCONHCH₂CH₂CH₂N=, -CH₂- 14 et -(CH)-H 6) ;1,48 (s, 3H: -CH₃ 19) ; 1,6 (s, 3H: -CH₃ 18) ; 2,1 (s, 3H, -COCH₃) ; 2,28 (mt, 1H : -(CH)-H 6) ; 2,78 (s, 3H : -NCH₃) ; 2,8 à 3,1 et 3,42 (mt et mf, respectivement 4H et 8H : -OCONHCH₂CH₂CH₂N(CH₂CH₂)₂NCH₃) ; 3,57 (d, 1H, J = 7 : -H 3) ; 4,01 et 4,1 (2d, 1H chacun, J = 8 : -CH₂- 20 et -H 7) ; 4,3 à 4,4 (-H 2′: signal masqué par la bande du solvant) ; 4,73 (d, 1H, J = 7 : -H 3′) ; 4,85 (d large, 1H, J = 9 : -H 5) ; 5,02 (mt, 1H : -H 7) ; 5,24 (s, 1H : -H 10) ; 5,32 (d, 1H, J = 7 : -H 2) ; 5,82 (t, 1H, J = 9 : -H 13) ; 7,02 [t, 1H, J = 8 : -C₆H₅ 3′(-H 4)] ; 7,12 [d, 2H, J = 8 : -C₆H₅ 3′ (-H 2 et -H 6)] ; 7,22 [t, 2H, J = 8 : -C₆H₅ 3′(-H 3 et -H 5)] ; 7,41 [t, 2H, J = 8 : -OCOC₆H₅ (-H 3 et -H 5)] ; 7,52 [t, 1H, J = 8 -OCOC₆H₅ (-H 4)] ; 7,8 [d, 2H, J = 8 : -OCOC₆H₅ (-H 2 et -H 6)].

### EXEMPLE 4

En opérant d'une manière analogue à celle décrite à l'exemple 2, mais à partir de 6,95 g de tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α et de 0,88 cm3 de morpholino-3 propylamine, on obtient après purification par chromatographie liquide haute performance avec comme phase mobile le mélange méthanol-(propanol-2)-hexane (20-5-75 en volumes) :
- 1,53 g de tert-butoxycarbonylamino-3 hydroxy-2 phényl-3 propionate- (2R,3S) d'acétoxy-4 benzoyloxy-2α époxy-5β,20 hydroxy-1 [(morpholino-3 propyl) carbamoyloxy]-10β oxo-9 (trichloro-2,2,2 éthoxy) carbonyloxy-7β taxène-11 yle-13α sous forme d'une meringue blanche,
- 1,35 g de tert-butoxycarbonylamino-3 hydroxy-2 phényl-3 propionate- (2R,3S) d'acétoxy-4 benzoyloxy-2α époxy-5β,20 hydroxy-1 [(morpholino-3 propyl) carbamoyloxy]-7β oxo-9 (trichloro-2,2,2 éthoxy) carbonyloxy-10β taxène-11 yle-13α sous forme d'une meringue blanche.

Le tert-butoxycarbonylamino-3 hydroxy-2 phényl-3 propionate-(2R,3S) d'acétoxy-4 benzoyloxy-2α époxy-5β,20 hydroxy- 1 [(morpholino-3 propyl) carbamoyloxy]-10β oxo-9 (trichloro-2,2,2 éthoxy) carbonyloxy-7β taxène-11 yle-13α est transformé en tert-butoxycarbonylamino-3 hydroxy-2 phényl-3 propionate-(2R,3S) d'acétoxy-4 benzoyloxy-2α époxy-5β,20 dihydroxy-1,7β [(morpholino-3 propyl) carbamoyloxy]-10β oxo-9 taxène-11 yle-13α par action du zinc dans un mélange de méthanol et d'acide acétique comme décrit à l'exemple 2 pour le tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxyl-10β oxo-9 (trichloro2,2,2 éthoxy) carbonyloxy-7β taxène-11 yle-13α.

Le produit ainsi obtenu possède les caractéristiques suivantes :
- spectre de RMN (400 MHz; CDCl₃)
δ (ppm) : 1,15 (s, 3H: -CH₃ 16 ou 17) ; 1,28 (s, 3H: -CH₃ 16 ou 17) ; 1,35 (s, 9H: -C(CH₃)₃) ; 1,68 (s, 3H : -CH₃ 19) ; 1,65 à 1,9 (mt, 2H : -OCONHCH₂CH₂CH₂N=) ; 1,88 (s, 3H : -CH₃ 18) ; 1,9 (mt, 1H : -(CH)- H 6) ; 2,28 (mt, 2H : -CH₂- 14) ; 2,4 (s, 3H : -COCH₃) ; 2,45 à 2,75 [mt, 7H : - CH₂N(CH₂CH₂)₂O et -(CH)- H 6] ; 3,28 et 3,42 (2 mt, 1H chacun : -OCONHCH₂CH₂CH₂N=) ; 3,8 [mt, 5H : -CH₂N(CH₂CH₂)₂O et -H 3] ; 4,18 et 4,31 (2d, 1H chacun, J = 8,5 : -CH₂- 20) ; 4,44 (dd, 1H, J = 11 et 7 : -H 7) ; 4,63 (s large, 1H : -H 2′) ; 4,98 (d large, 1H, J = 10 : -H 5) ; 5,27 (d large, 1H, J = 9 : -H 3′) ; 5,41 [d, 1H, J = 9 : :NHCOOC(CH₃)₃] ; 5,67 (d, 1H, J = 7 : -H 2) ; 6,21 (s, 1H : -H 10) ; 6,25 (mt, 1H : -H 13) ; 6,58 (t large, 1H, J = 5 : -NHCH₂CH₂CH₂N=) ; 7,25 à 7,45 (mt, 5H : -C₆H₅ 3′) ; 7,52 [t, 2H, J = 8 : -OCOC₆H₅ (-H 3 et -H 5)] ; 7,62 [t, 1H, J = 8: -OCOC₆H₅ (-H 4)]; 8,12 [d, 2H, J = 8: -OCOC₆H₅ (-H 2 et -H 6)].

Le tert-butoxycarbonylamino-3 hydroxy-2 phényl-3 propionate-(2R,3S) d'acétoxy-4 benzoyloxy-2α époxy-5β,20 hydroxy-1 [(morpholino-3 propyl) carbamoyloxyl -7β oxo-9 (trichloro-2,2,2 éthoxy) carbonyloxy-10β taxène-11 yle-13α est transformé en tert-butyoxycarbonylamino-3 hydroxy-2 phényl-3 propionate- (2R,3S) d'acétoxy-4 benzoyloxy-2α époxy-5β,20 dihydroxy-1,10β [(morpholino-3 propyl) carbamoyloxy]-7β oxo-9 taxène-11 yle-13α par action du zinc dans un mélange de méthanol et d'acide acétique comme décrit à l'exemple 2 pour le tert-butoxycarbonylamino-3 hydroxy-2 phényl-3 propionate-(2R,3S) d'acétoxy-4 benzoyloxy-2α époxy-5β,20 hydroxy-1 [(diméthylamino-3 propyl) carbamoyloxy]-10β oxo-9 (trichloro-2,2,2 éthoxy) carbonyloxy-7β taxène-11 yle-13α.

Le produit ainsi obtenu possède les caractéristiques suivantes :
- spectre de RMN (400 MHz ; CDCl₃)
δ (ppm) : 1,11 (s, 3H: -CH₃ 16 ou 17) ; 1,24 (s, 3H: -CH₃ 16 ou 17) ; 1,37 (s, 9H: -C(CH₃)₃) ; 1,6 à 1,9 (mt, 2H: -OCONHCH₂CH₂CH₂N=) ; 1,81 (s, 3H: -CH₃ 19) ; 1,93 (s, 3H : -CH₃ 18) ; 1,93 (mt, 1H : -(CH)-H 6) ; 2,3 (d, 2H, J = 8 : -CH₂- 14) ; 2,4 (s, 3H: -COCH₃) ; 2,4 à 2,7 [mt, 7H : -CH₂N(CH₂CH₂)₂O et -(CH)-H 6] ; 3,12 et 3,31 (2 mt, 1H chacun: -OCONHCH₂CH₂CH₂N=) ; 3,5 (mf, 1H : -OH 2′) ; 3,8 [mf, 4H: -CH₂N(CH₂CH₂O] ; 4,0 (d, 1H, J = 7: -H 3) ; 4,2 et 4,33 (2d, 1H chacun, J = 8,5 : -CH₂- 20) ; 4,64 (mf, 1H : -H 2′) ; 4,94 (d large, 1H, J = 9,5 : -H 5) ; 5,28 (d large, 1H, J = 9 : -H 3′) ; 5,38 (dd, 1H, J = 10 et 7 : -H 7) ; 5,45 [d large, 1H, J = 9 : -NHCOOC(CH₃)₃] ; 5,51 (s, 1H : -H 10) ; 5,68 (d, 1H, J = 7 : -H 2) ; 5,8 (t, 1H, J = 5 : -NHCH₂CH₂CH₂N=) ; 6,21 (t, 1H, J = 8 : -H 13) ; 7,25 à 7,45 (mt, 5H : -C₆H₅ 3′) ; 7,51 [t, 2H, J = 8 : -OCOC₆H₅ (-H 3 et -H 5)] ; 7,62 [t, 1H, J = 8 : -OCOC₆H₅ (-H 4)] ; 8,12 [d, 2H, J = 8: -OCOC₆H₅ (-H 2 et -H 6)].

### EXEMPLE 5

A une solution de 0,29 g de tert-butoxycarbonylamino-3 hydroxy-2 phényl-3 propionate-(2R,3S) d'acétoxy-4 benzoyloxy-2α époxy-5β,20 hydroxy-1 oxo-9 bis(trichloro-2,2,2 éthoxy) carbonyloxy-7β,10β taxène-11 yle-13α dans 100 cm3 d'acétonitrile, maintenue sous atmosphère d'argon, on ajoute 0,31 cm3 de diméthylamino-3 propylamine. Le milieu réactionnel est chauffé sous agitation pendant 3 heures à une température voisine de 60°C puis refroidi à une température voisine de 20°C et concentré à sec sous pression réduite (2,7 kPa) à 40°C. On obtient 0,32 g d'une meringue blanche que l'on purifie par chromatographie sur 30 g d'alumine, (0,12-0,15 mm) contenus dans une colonne de 1,5 cm de diamètre [éluant : dichlorométhane-méthanol (95-5 en volumes)] en recueillant des fractions de 10 cm3. Les fractions 7 à 15 sont réunies et concentrées à sec sous pression réduite (2,7 kPa) à une température voisine de 40°C. On obtient ainsi 0, 12 g de tert-butoxycarbonylamino-3 hydroxy-2 phényl-3 propionate-(2R,3S) d'acétoxy-4 benzoyloxy-2α époxy-5β,20 hydroxy-1 bis-[(diméthylamino-3 propyl) carbamoyloxyl-7β,10β oxo-9 taxène-11 yle-13α sous forme d'une meringue blanche dont les caractéristiques sont les suivantes:
- pouvoir rotatoire: [α]²⁰_{D} = -26° (c = 0,75; méthanol)
- spectre de RMN (400 MHz; CDCl₃)
   δ (ppm) : 1,17 (s, 3H : -CH₃ 16 ou 17) ; 1,20 (s, 3H: -CH₃ 16 ou 17) ; 1,36 (s, 9H : C(CH₃)₃) ; 1,6 à 1,8 [mt, 4H : -NHCH₂CH₂CH₂N(CH₃)₂] ; 1,78 (s, 3H : -CH₃ 19) ; 1,83 (mt, 1H : -(CH)-H 6) ; 1,96 (s, 3H : -CH₃ 18) ; 2,26 [s, 6H : -N(CH₃)₂] ; 2,36 (s, 3H : -COCH₃) ; 2,2 à 2,6 [mt, 6H : -CH₂- 14 et -CH₂N(CH₃)₂] ; 2,66 (mt, 1H : -(CH)-H 6) ; 3,24 [mt, 4H : -NHCH₂CH₂CH₂N(CH₃)₂] ; 3,92 (d, 1H, J = 7: -H 3) ; 4,16 et 4,3 (2d, 1H chacun, J = 8 : -CH₂- 20) ; 4,62 (s large, 1H : -H 2′) ; 4,93 (d, 1H, J = 9 : - H 5) ; 5,27 (mt, 1H : -H 3′) ; 5,4 (mt, 1H : -H 7 ) ; 5,48 et 5,76 [2 mt, 1H chacun: -NHCH₂CH₂CH₂N(CH₃)₂] ; 5,55 [d large, 1H: -NHCOOC(CH₃)₃] ; 5,64 (d, 1H, J = 7 : -H 2) ; 6,16 (t large, 1H, J = 9 : -H 13) ; 6,32 (s, 1H : -H 10) ; 7,3 à 7,5 (mt, 5H : -C₆H₅ 3′) ; 7,5 [t, 2H, J = 7,5 : -OCOC₆H₅ (-H 3 et -H 5)] ; 7,61 [t, 1H, J = 7,5: -OCOC₆H₅(-H 4)] ; 8,1 [d, 2H, J = 7,5: -OCOC₆H₅(-H 2 et -H 6)].

A une solution de 10,6 mg de tert-butoxycarbonylamino-3 hydroxy-2 phényl-3 propionate-(2R,3S) d'acétoxy-4 benzoyloxy-2α époxy-5β,20 hydroxy-1 bis[(diméthylamino-3 propyl) carbamoyloxyl-7β,10β oxo-9 taxène-11 yle-13α précédemment obtenus, dans 0,2 cm3 d'une solution aqueuse 0,1 N d'acide chlorhydrique, on ajoute 0,4 cm3 d'eau distillée et lyophilise la solution obtenue. On obtient ainsi 10 mg de dichlorhydrate de tert-butoxycarbonylamino-3 hydroxy-2 phényl-3 propionate-(2R,3S) d'acétoxy-4 benzoyloxy-2α époxy-5β,20 hydroxy-1 bis-[(diméthy1amino-3 propyl) carbamoyloxyl-7β,10β oxo-9 taxène-11 yle-13α dont les caractéristiques sont les suivantes :
- spectre de RMN (400 MHz; D₂O/CH₃COOD 90/10 en volumes)
δ (ppm) : 0,85 (s, 3H : -CH₃ 16 ou 17) ; 0,9 (s, 3H : -CH₃ 16 ou 17) ; 1,10 (bs, 9H : -C(CH₃)₃) ; 1,5 (s, 3H : -CH₃ 19) ; 1,6 à 1,8 [m, 10H : -CH₃ 18, -NHCH₂CH₂CH₂N(CH₃)₂, -CH₂, 14, -(CH)-H 6] ; 2,10 (s, 3H : -COCH₃) ; 2,3 (m, 1H : -(CH)- H 6) ; 2,65 [2s, 6H chacun : -N(CH₃)₂] ; 2,8 à 3,1 [vbm, 8H : -CH₂N(CH₃)₂, -NHCH₂CH₂CH₂N(CH₃)2]; 3,6 (bd, 1H: -H 3) ; 4 et 4,15 (2d, AB, 2H : -CH₂- 20) ; 4,40 (bd, 1H : -H 2′) ; 4,70 (vbs, 1H : -H 3′) ; 4,90 (bd, 1H : -H 5) ; 5,10 (bdd, 1H : -H 7) ; 5,35 (bd, 1H : -H 2) ; 5,8 (bt, 1H : -H 13) ; 6,05 (s, 1H : -H 10) ; 7,0 à 7,25 (mt, 5H : -C₆H₅ 3′) ; 7,4 [t, 2H, J = 7,5 : -OCOC₆H₅(-H 3 et -H 5)] ; 7,5 [t, 1H, J = 7,5 : -OCOC₆H₅(-H 4)] ; 7,80 [d, 2H, J = 7,5 : -OCOC₆H₅(-H 2 et -H 6)].

### EXEMPLE 6

En opérant d'une manière analogue à celle décrite à l'exemple 5, mais à partir de 1,18 g de tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α et de 1,6 g de (méthyl-4 pipérazinyl)-3 propylamine on obtient 1,1 g de tert-butoxycarbonylamino-3 hydroxy-2 phényl-3 propionate-(2R,3S) d'acétoxy-4 benzoyloxy-2α époxy-5β,20 hydroxy-1 bis{[(méthyl-4 pipérazinyl)-3 propyl] carbamoyloxy}-7β,10β oxo-9 taxène-11 yle-13α sous forme d'une meringue blanche dont les caractéristiques sont les suivantes :
- pouvoir rotatoire : [α]²⁰_{D} = -22,9° (c = 0,51 ; méthanol)
- spectre de RMN (400 MHz; CDCl₃)
   δ (ppm) : 1,24 (s, 3H : -CH₃ 16 ou 17) ; 1,28 (s, 3H : -CH₃ 16 ou 17 ) ; 1,4 (s, 9H : -C(CH₃)₃) ; 1,6 à 1,85 (mt, 4H : -OCONHCH₂CH₂CH₂N=) ; 1,83 (s, 3H : -CH₃ 19) ; 1,87 (ddd, 1H, J = 15, 11 et 2: -(CH)-H 6) ; 2,01 (s, 3H : -CH₃ 18) ; 2,3 à 2,7 [mt, 31H: -CH₂N(CH₂CH₂)₂NCH₃ -COCH₃ et -CH₂- 14] ; 2,7 (mt, 1H: -(CH)-H 6) ; 3,15 et 3,45 (mt, 4H : -OCONHCH₂CH₂CH₂N=) ; 4,0 (d, 1H, J = 7 : -H 3) ; 4,21 et 4,32 (2d, 1H chacun, J = 8 : -CH₂- 20) ; 4,66 (d, 1H, J = 2 : -H 2′) ; 4,98 (d large, 1H, J = 9 : -H 5) ; 5,28 (mt, 1H : -H 3′) ; 5,4 à 5,5 [mt, 2H : -H 7 et -NHCOOC(CH₃)₃] ; 5,7 (d, 1H, J = 7 : -H 2) ; 6,0 et 6,4 (2 mf, 1H chacun : -NHCH₂CH₂CH₂N=) ; 6,21 (t, 1H, J = 9 : -H 13) ; 6,4 (s, 1H : -H 10) ; 7,3 à 7,5 (mt, 5H : -C₆H₅ 3′) ; 7,51 [t, 2H, J = 7,5 : -OCOC₆H₅ (-H 3 et -H 5)] ; 7,64 [t, 1H, J = 7,5 : -OCOC₆H₅(-H 4)] ; 8,12 [d, 2H, J = 7,5: -OCOC₆H₅ (-H 2 et -H 6)].

Une solution de 0,35 g de tert-butoxycarbonylamino-3 hydroxy-2 phényl-3 propionate-(2R,3S) d'acétoxy-4 benzoyloxy-2α époxy-5β,20 hydroxy-1 bis-{[(méthyl-4 pipérazinyl)-3 propyl] carbamoyloxy}-7β,10β oxo-9 taxène-11 yle-13α précédemment obtenus, dans 12 cm3 d'une solution aqueuse 0,1N d'acide chlorhydrique est lyophillisée. On obtient ainsi 0,365 g de tétrachlorhydrate de tert-butoxycarbonylamino-3 hydroxy-2 phényl-3 propionate-(2R,3S) d'acétoxy-4 benzoyloxy-2α époxy-5β,20 hydroxy-1 bis-{[(méthyl-4 pipérazinyl)-3 propyl] carbamoyloxy}-7β,10β oxo-9 taxène-11 yle-13α dont les caractéristiques sont les suivantes :
- pouvoir rotatoire: [α]²⁰_{D} = -22° (c = 0,41 ; méthanol)
- spectre de RMN (400 MHz ; D₂O)
   δ (ppm) : 1,1 (s, 3H : -CH₃ 16 ou 17) ; 1,14 (s, 3H : -CH₃ 16 ou 17) ; 1,36 (s, 9H : -C(CH₃)₃) ; 1,72 et 1,92 (2s, 3H chacun : -CH₃ 18 et -CH₃ 19) ; 1,65 à 2,05 (mt, 7H: -OCONHCH₂CH₂CH₂N=, -CH₂- 14 et - (CH)-H 6 ) ; 2,38 (s, 3H : -COCH₃) ; 2,55 (mt, 1H : -(CH)-H 6) ; 2,92 et 2,94 (2s, 6H : -NCH₃) ; 3,0 à 3,7 (mt, 24H : -OCONHCH₂CH₂CH₂N(CH₂CH₂)₂NCH₃) ; 3,79 (d, 1H, J = 7: -H 3) ; 4,23 et 4,4 (2d, 1H chacun, J = 9 : -CH₂- 20) ; 4,67 (d, 1H, J = 7: -H 2′) ; 4,98 (mf, 1H : -H 3′) ; 5,16 (d large, 1H, J = 9: -H 5) ; 5,33 (dd, 1H, J = 11 et 7: - H 7) ; 5,58 (d, 1H, J = 7: -H 2) ; 6,07 (t, 1H, J = 9 : -H 13) ; 6,28 (s, 1H : -H 10) ; 7,27 [t, 1H, J = 7,5 : -C₆H₅ 3′ (-H 4)] ; 7,38 [d, 2H, J = 7,5 : -C₆H₅ 3′(-H 2 et -H 6)] ; 7,48 [t, 2H, J = 7,5 : -C₆H₅ 3′(-H 3 et -H 5)] ; 7,67 [t, 2H, J = 7,5 : -OCOC₆H₅ (-H 3 et -H 5)] ; 7,78 [t, 1H, J = 7,5 : -OCOC₆H₅ (-H 4)] ; 8,18 [d, 2H, J = 7,5 : -OCOC₆H₅ (-H ₂ et -H 6)].

### EXEMPLE 7

En opérant d'une manière analogue à celle décrite à l'exemple 5, mais à partir de 0,58 g de tert-butoxycarbonylamino-3 hydroxy-2 phényl-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α et de 0,73 cm3 de morpholino -3 propylamine, on obtient 0,4 g de tert-butoxycarbonylamino-3 hydroxy-2 phényl-3 propionate-(2R,3S) d'acétoxy-4 benzoyloxy-2α époxy-5β,20 hydroxy-1 bis-[(morpholino-3 propyl) carbamoyloxy-7β,10β oxo-9 taxène-11 yle-13α sous forme d'une meringue blanche dont les caractéristiques sont les suivantes :
- pouvoir rotatoire: [α]²⁰_{D} = -24,7° (c = 0,52; méthanol)
- spectre de RMN (400 MHz; CDCl₃)
   δ (ppm) : 1,25 (s, 3H : -CH₃ 16 ou 17) ; 1,28 (s, 3H : -CH₃ 16 ou 17) ; 1,41 (s, 9H : -C(CH₃)₃) ; 1,6 à 1,85 (mt, 4H : -OCONHCH₂CH₂CH₂N=) ; 1,81 (s, 3H : -CH₃ 19) ; 1,89 (ddd, 1H, J = 15, 11 et 2 : -(CH)-H 6) ; 2,0 (s, 3H : -CH₃ 18) ; 2,36 (mt, 2H: -CH₂- 14) ; 2,36 (s, 3H : -COCH₃) ; 2,4 à 2,6 [mt, 12H : -CH₂N(CH₂)₂O] ; 2,7 (mt, 1H : -(CH)-H 6) ; 3,15 à 3,45 (mt, 4H: -OCONHCH₂CH₂CH₂N=) ; 3,76 [mt, 8H: -CH₂N(CH₂CH₂)₂O] ; 3,98 (d, 1H, J = 7: -H 3) ; 4,20 et 4,31 (2d, 1H chacun, J = 8 : -CH₂- 20) ; 4;65 (d, 1H, J = 2 : -H 2′) ; 4,98 (d large, 1H, J = 10 : -H 5) ; 5,3 (d large, 1H, J = 9: H 3′) ; 5,4 [d, 1H, J = 9: -NHCOOC(CH₃)₃] ; 5,45 (mt, 1H: -H 7) ; 5,7 (d, 1H, J = 7: -H 2) ; 5,6 à 6,2 (mf, 2H : -NHCH₂CH₂CH₂N=) ; 6,22 (t, 1H, J = 9 : -H 13) ; 6,38 (s, 1H : -H 10) ; 7,3 à 7,5 (mt, 5H : -C₆H₅ 3′) ; 7,51 [t, 2H, J = 7,5 : -OCOC₆H₅(-H 3 et -H 5)] ; 7,62 [t, 1H, J = 7,5 : -OCOC₆H₅(-H 4)] ; 8,11 [d, 2H, J = 7,5 : -OCOC₆H₅(-H 2 et -H 6)].

A une solution de 11,5 mg de tert-butoxycarbonylamino-3 hydroxy-2 phényl-3 propionate-(2R,3S) d'acétoxy-4 benzoyloxy-2α époxy-5β,20 hydroxy-1 bis[morpholino-3 propyl) carbamoyloxyl-7β,10β oxo-9 taxène-11 yle-13α précédemment obtenu, dans 0,2 cm3 d'une solution aqueuse 0,1N d'acide chlorhydrique, on ajoute 0,4 cm3 d'eau distillée et lyophilise la solution obtenue. On obtient ainsi 12 mg de dichlorhydrate de tert-butoxycarbonylamino-3 hydroxy-2 phényl-3 propionate-(2R,3S) d'acétoxy-4 benzoyloxy-2α époxy-5β,20 hydroxy-1 bis-[morpholino-3 propyl) carbamoyloxyl-7β,10β oxo-9 taxène-11 yle-13α dont les caractéristiques sont les suivantes :
- spectre de RMN (400 MHz ; D₂O/CH₃COOD: 90/10 en volumes)
δ (ppm) : 0,85 (s, 3H : -CH₃ 16 ou 17) ; 0,9 (s, 3H : -CH₃ 16 ou 17) ; ,1,1 (s, 9H : -C(CH₃)₃) ; 1,5 (s, 3H : -CH₃ 19 ) ; 1,6 à 1,8 (m, 10H : -CH₃ 18, -OCONHCH₂CH₂CH₂N=, -CH₂- 14, -(CH)-H 6) ; 2;1 (s, 3H : -COCH₃) ; 2,3 (m, 1H : -(CH)-H 6) ; 2,8 à 3,10 (vbm, 12H : -CH₂N(CH₂CH₂)₂O) ; 3,3 (bm, 4H : -OCONHCH₂CH₂CH₂N=) ; 3,6 (bm, 4H : -CH₂N(CH₂CH₂)₂O) ; 3,85 (bm, 4H : -CH₂N(CH₂CH₂)₂O) ; 4 et 4,15 (bd, AB, 2H : -CH₂- 20 ) ; 4,4 (bd, 1H : -H 2′) ; 4,75 (bs, 1H : -H 3′) ; 4,9 (bd, 1H : -H 5) ; 5,1 (bdd, 1H : -H 7) ; 5,35 (bd, 1H : -H 2) ; 5,8 (bt, 1H : -H 13) ; 6,04 (s, 1H : -H 10) ; 7,0 à 7,25 (m, 5H : -C₆H₅ 3′) ; 7,4 [t, 2H, J = 7,5 : -OCOC₆H₅ (-H 3 et -H 5)] ; 7,52 [t, 1H, J = 7,5 : -OCOC₆H₅ (-H 4)] ; 7,8 [d, 2H, J = 7,5 : -OCOC₆H₅ (-H 2 et -H 6)].

Les nouveaux produits de formule générale (Ia) présentent des activités biologiques particulièrement intéressantes.

Les nouveaux produits de formule générale (Ia) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et,/ou organes comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (Ia) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émusifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. Les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérélisation pot être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques , des anticorps monoclonaux, des thérapies immunologiques ou des radiothérapies ou des modificateurs des réponses biologiques. Les modificateurs des réponses incluent, de manière non limitative, les lymphokines et les cytokines telles que les intérleukines, les interférons(α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomusine, la sémustine et la streptozocine, les triazènes comme la dacarbazine, les antimétabolites comme les analogues de l'acide folique tel que le méthotrexate, les analogues de pyrimidine comme le fluororacil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination du platine tel que le cisplatine, les urées substituées tel que l'hydroxyurée, les dérivés de méthylhydrazine comme la procarbazine, les suppresseurs adrénocoticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogène comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire d'administrer des doses d'entretien 1 à 8 fois par jour, de préférence 1 à 4 fois, selon les besoins physiologiques du malade considéré. 11 est aussi possible que pur certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0, 1 et 100 mg/kg et, de préférence entre 0,5 et 50 mg/kg et, encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

### EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par introduction dans une perfusion d'un soluté physiologique pendant 1 heure.

## Revendications

**1 -** Nouveaux dérivés d'analogues du taxol de formule générale : 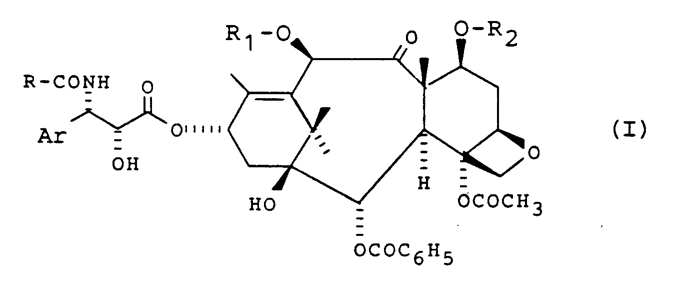 dans laquelle :
Ar représente un radical aryle,
R représente
- un radical de formule générale
R₇O- (II)
dans laquelle R₇ représente un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en-4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
- ou un radical hétérocyclyle azoté saturé ou non saturé contenant 4 ou 6 chaînons et éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone,
R₁ et R₂, identiques ou différents, représentent un atome d'hydrogène ou un radical de formule générale: 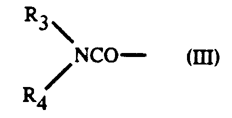 dans laquelle R₃ et R₄, identiques ou différents, représentent un atome d'hydrogène ou un radical alcoyle contenant 1 à 4 atomes de carbone en chaîne droite ou ramifiée éventuellement substitué :
a) par un radical hydroxy, carboxy, alcoyloxycarbonyle dont la partie alcoyle contient 1 à 4 atomes de carbone et est éventuellement substituée par un radical phényle,
b) par un radical de formule générale : 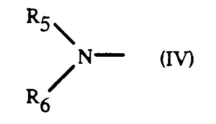 dans laquelle R₅ et R₆, identiques ou différents, représentent un atome d'hydrogène ou un radical alcoyle contenant 1 à 4 atomes de carbone en chaîne droite ou ramifiée, ou bien R₅ et R₆ forment ensemble avec l'atome d'azote auquel ils sont liés un hétéroycle saturé ou non saturé à 5 ou 6 chaînons contenant éventuellement un second hétéroatome choisi parmi les atomes d'azote (éventuellement substitué par un radical alcoyle contenant 1 à 4 atomes de carbone ou benzyle), d'oxygène ou de soufre, ou bien R₃ et R₄ forment ensemble avec l'atome d'azote auquel ils sont liés un hétérocycle saturé ou non saturé à 5 ou 6 chaînons contenant éventuellement un second hétéroatome choisi parmi les atomes d'azote (éventuellement substitué par un radical alcoyle contenant 1 à 4 atomes de carbone ou benzyle), d'oxygène ou de soufre,
étant entendu que l'un au moins des symboles R₁ ou R₂ représente un radical de formule générale (II) ainsi que, lorsqu'ils existent, leurs sels d'addition avec les acides.

**2 -** Procédé de préparation des nouveaux dérivés selon la revendication 1 caractérisé en ce que l'on fait agir une amine de formule générale: 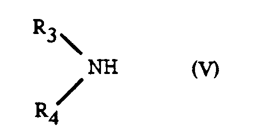 dans laquelle R₃ et R₄ sont définis comme dans la revendication 1 sur un dérivé du taxane de formule générale: 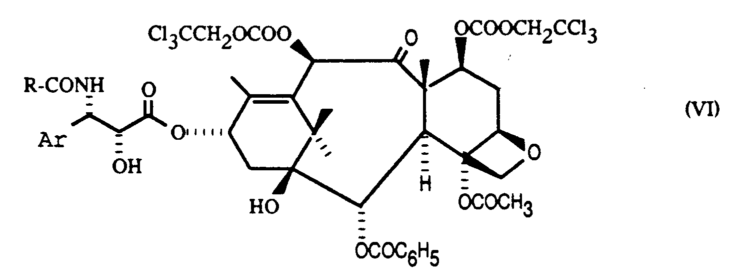 dans laquelle R et Ar sont définis comme dans la revendication 1, pour obtenir un produit de formule générale: 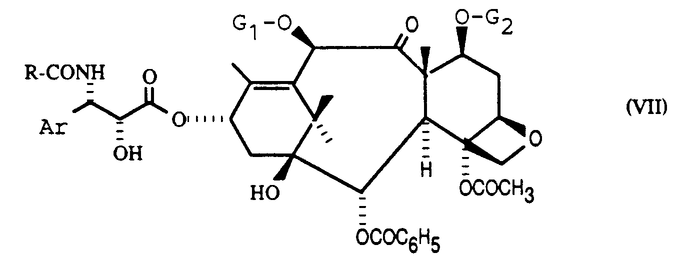 dans laquelle R, Ar sont définis comme précédemment, G₁ et G₂ représentent chacun un radical de formule générale (II) ou un groupement protecteur (CCl₃CH₂OCO-), étant entendu que l'un au moins des radicaux G₁ et G₂ représente un radical de formule générale (II), suivie, si nécessaire, du remplacement du ou des groupements protecteurs (CCl₃CH₂OCO-) par un atome d'hydrogène. sépare les produits de formule générale (I) de leur mélange et isole le produit obtenu éventuellement sous forme de sel.

**3 -** Procédé selon la revendication 2 caractérisé en ce que l'on fait réagir l'amine de formule générale (I) sur le dérivé du taxane de formule générale (III) en opérant dans un solvant organique inerte tel qu'un hydrocarbure aliphatique halogéné comme le chlorure de méthylène à une température comprise entre 0°C et la température d'ébullition du mélange réactionnel.

**4 -** Procédé selon la revendication 3 caractérisé en ce que le remplacement du groupement protecteur trichloro-2,2,2 éthoxycarbonyle par un atome d'hydrogène est effectué au moyen de zinc dans l'acide acétique éventuellement en présence de méthanol à une température comprise entre 30 et 80°C.

**5 -** Composition pharmaceutique caractérisée en ce qu'elle contient une quantité suffisante d'un produit selon la revendication 1 en association avec un ou plusieurs diluants ou adjuvants pharmaceutiquement acceptables inertes ou pharmacologiquement actifs.
